Europäisches Patentamt

European Patent Office

Office européen des brevets

(19)

(11) **EP 0 468 847 B1**

(12) **FASCICULE DE BREVET EUROPEEN**

(45) Date de publication et mention
de la délivrance du brevet:
**16.10.1996 Bulletin 1996/42**

(51) Int. Cl.$^6$: **A61F 7/00**

(21) Numéro de dépôt: **91401936.9**

(22) Date de dépôt: **01.07.1991**

(54) **Appareil d'hyperthermie ultrasonore extracorporelle ultrarapide**

Schnelles, extrakorporales Ultraschallhyperthermiegerät

Rapid ultrasonic extracorporal hyperthermia apparatus

(84) Etats contractants désignés:
**AT BE CH DE DK ES GB GR IT LI LU NL SE**

(30) Priorité: **23.07.1990 FR 9009717**

(43) Date de publication de la demande:
**29.01.1992 Bulletin 1992/05**

(73) Titulaire: **EDAP INTERNATIONAL**
**F-77200 Croissy Beaubourg-Marne la Vallée**
**(FR)**

(72) Inventeur: **Dory, Jacques**
**F-77450 Coupvray (FR)**

(74) Mandataire: **Hirsch, Marc-Roger et al**
**Cabinet Hirsch**
**34 rue de Bassano**
**75008 Paris (FR)**

(56) Documents cités:
**EP-A- 0 045 265      EP-A- 0 162 735**
**EP-A- 0 339 693      EP-A- 0 370 841**
**GB-A- 820 814        US-A- 3 735 755**

## Description

Il est connu, en particulier du brevet français No 84 06877, déposé le 3 Mai 1984, pour : "Appareil d'examen et de localisation de tumeurs par ultrasons muni d'un dispositif de traitement localisé par hyperthermie", d'utiliser un faisceau ultrasonore focalisé pour provoquer un échauffement très localisé des tissus biologiques en vue de détruire les tumeurs.

Dans l'appareil décrit dans le brevet susvisé, l'émission de faisceau se fait par trains d'ondes à haute fréquence (0,5 à 5 MHz par exemple, les fréquences les plus basses étant utilisées pour la destruction des structures les plus profondes à l'intérieur du corps) et avec une puissance de crête relativement faible (une dizaine à une centaine de watts, les puissances les plus élevées étant utilisées pour la destruction des structures les plus profondes).

Ces trains d'ondes sont séparés par des intervalles pendant lesquels il est possible d'effectuer une échographie en temps réel, en général du type B permettant, soit d'effectuer une relocalisation du foyer par rapport à la cible (laquelle subit les mouvements naturels des tissus sous l'influence de la respiration), soit d'examiner les soit d'examiner les altérations subies par les tissus dans la zone traitée.

Avec la puissance et les fréquences utilisées - qui sont fonction de la profondeur de la cible - la température de la cible est portée à 45°C environ, température suffisante en principe pour détruire les cellules malignes, et l'on a pensé jusqu'ici qu'une élévation de température excessive de la cible pourrait provoquer des brûlures graves dans la zone qui l'entoure, du fait de la diffusion thermique.

Il en résulte que les temps de traitement sont relativement longs, leur durée pouvant atteindre plusieurs dizaines de minutes, voire plusieurs heures.

Dans le document EP-A-0 370 841, est décrit un dispositif de traitement par ultrasons utilisant une céramique piézoélectrique focalisante et oscillante. Ce document propose d'utiliser des fréquences de traitement de l'ordre de 5 à 10 MHz, avec des puissances moyennes de l'ordre de 1 kW.

Le document US-A-3 735 755 décrit un procédé et un appareil de chirurgie non-invasive. Ce document donne, en fonction de la durée d'impulsion, l'intensité acoustique nécessaire pour produire des lésions dans la substance blanche du cerveau des mammifères.

Le document GB-B-820 814 décrit un appareil de traitement des tissus par ultrasons. Ce document propose d'utiliser des fréquences de traitement de l'ordre de 1 MHz, avec des intensité de l'ordre de 10 à 1000 W/cm$^2$.

Ces documents n'indiquent pas précisément, comme le fait l'invention, comment déterminer les paramètres qui définissent la concentration globale du faisceau ultrasonore dans la tache focale et la puissance, de façon à optimiser la durée du traitement, et limiter les brûlures dans la zone qui entoure la cible.

L'invention repose sur la découverte du fait que la multiplication de puissance de crête des ondes utilisées par un facteur allant de 10 à 100 en fonction de la profondeur et du pouvoir absorbant de la cible permet au contraire, en provoquant une élévation de température ultrarapide, de réduire notablement les effets de la diffusion thermique et d'aboutir à une destruction de la cible en des temps de l'ordre de la seconde.

L'invention propose un appareil d'hyperthermie ultrasonore extracorporelle, permettant l'émission focalisée de trains d'ondes ultrasonores à différentes profondeurs, caractérisé en ce que les dits trains d'ondes ultrasonores présentent une fréquence comprise entre 0,5 et 10 MHz et des puissances électriques de crête comprises entre 10 kW et quelques centaines de watts, les paramètres qui définissent la concentration globale du faisceau ultrasonore dans la tache focale et la puissance étant déterminés pour que, quelle que soit la profondeur et la nature des tissus, le temps de l'émission de traitement soit de l'ordre de celui qui permet d'atteindre une température de destruction de la cible pendant la partie linéaire de la courbe d'élévation de température de celle-ci en fonction du temps.

Avantageusement, la fréquence ainsi que le diamètre de la surface émettrice dudit appareil et celui de la tache focale sont déterminés en fonction de la nature et de la profondeur de la cible, pour que le facteur de concentration globale du faisceau soit maximal et la puissance est alors déterminée pour une valeur donnée de la fréquence et des diamètres sélectionnés, pour que la destruction de la cible s'effectue à une température voisine de 58°C, en un temps compris entre 0,5 et 3 sec.

Les essais effectués par la Demanderesse ont permis de constater que cet agencement de l'appareil, lequel requiert la mise en oeuvre de moyens appropriés à l'émission quasi-continue de puissances de crête très élevées, permet de minimiser la destruction des cellules saines tout en augmentant l'efficacité de la destruction de la cible, en particulier par un effet supplémentaire de destruction mécanique des cellules de celles-ci, et aboutit ainsi à une nouvelle technique d'hyperthermie ultrasonore localisée qui justifie le terme d'hyperthermie ultrarapide qui la désignera ci-après.

Un autre avantage de l'hyperthermie ultrarapide est qu'elle permet d'améliorer considérablement l'examen échographique des altérations de la cible au cours du traitement.

Suivant un autre aspect de l'invention, cet examen est effectué par échographie A ou B pendant des interruptions de l'onde de traitement effectuées à une cadence déterminée pour que l'échogramme ou respectivement l'image aient eu le temps de subir des modifications détectables (ce qui pourra être le cas en quelques dixièmes de secondes en hyperthermie ultrarapide), non masquées par les modifications parasites provoquées par le mouvement des trains (comme c est en pratique le cas dans l'hyperthermie de l'art antérieur).

L'invention porte par ailleurs sur des techniques particulières d'échographie A ou B qui facilitent la détection ultrarapide des altérations subies par la cible et permettent ainsi d'arrêter le traitement dès sa destruction.

L'invention porte enfin sur un procédé de détermination des paramètres de réglages d'un appareil d'hyperthermie ultrasonore extracorporelle, comprenant les étapes consistant à :

- déterminer la distance focale à partir de la profondeur de la dite cible;
- choisir la fréquence des trains d'ondes ultrasonores dans une plage entre 0,5 et 10 MHz pour que la concentration globale de l'énergie ultrasonore soit maximale sur la tache focale, à la distance focale déterminée;
- déterminer expérimentalement la durée de la partie linéaire de la montée en température;
- choisir la puissance électrique de crête dans une plage entre quelques centaines de watts et 10 kW, pour atteindre une température de destruction de la cible pendant la partie linéaire de la courbe d'élévation de température.

D'autres particularités, ainsi que les avantages de l'invention apparaîtront clairement à la lumière de la description ci-après, dans laquelle les techniques de détection ultrarapide ont été décrites en premier, afin de facilité l'exposé ultérieur de l'agencement de l'appareil qui permet l'hyperthermie ultrarapide.

Au dessin annexé :

La figure 1 représente schématiquement un appareil d'hyperthermie ultrarapide muni de moyens de détection ultrarapide des altérations de la cible en cours de traitement ;

La figure 2 est un diagramme destiné a illustrer le fonctionnement desdits moyens de détection ; et

La figure 3 représente les courbes d'élévation de la température d'un tissu biologique en fonction du temps.

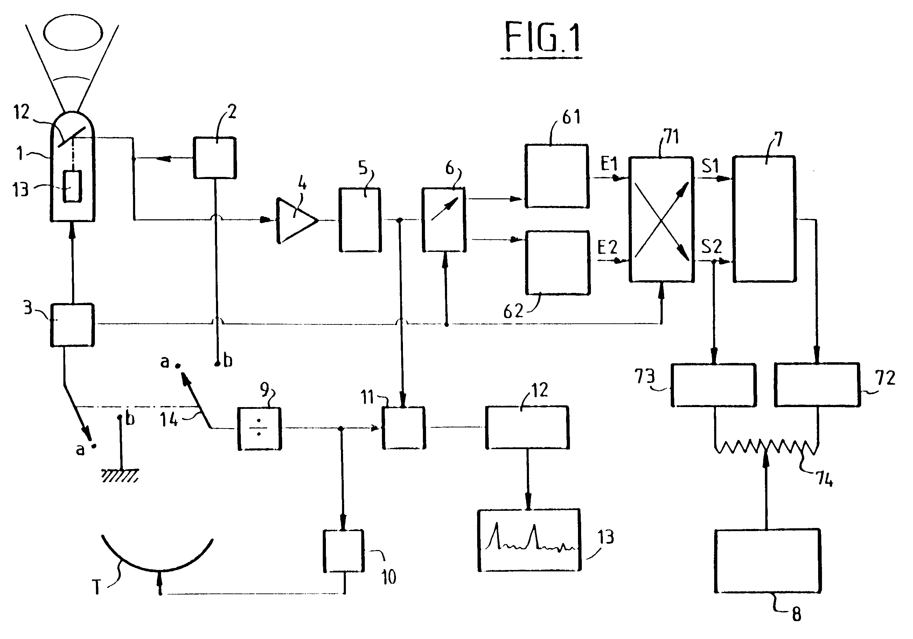

A la figure 1, on a représenté schématiquement un échographe de type connu comprenant une sonde temps réel 1 comportant un élément piézoélectrique 12 entraîné en oscillation par un moteur électrique 13, par l'intermédiaire d'un système de transmission symbolisé par une ligne en trait mixte.

A titre d'exemple, cette sonde peut être réalisée suivant la description donnée dans le brevet français No 80 16718, déposé le 29 Juillet 1980, pour : "Sonde d'échographie à balayage sectoriel mécanique".

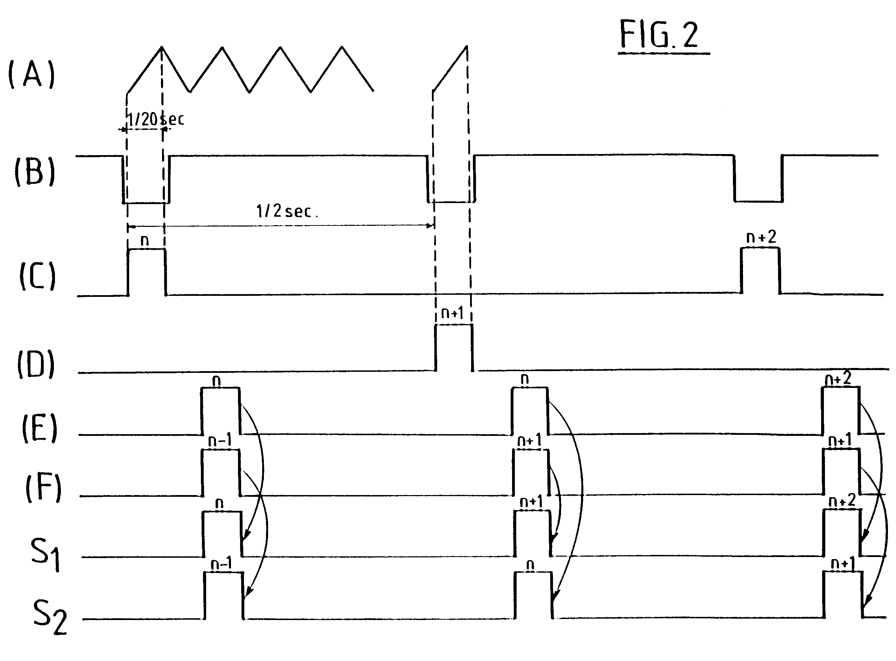

L'élément piézoélectrique 12 est excité par un émetteur d'impulsions 2 et le moteur est commandé par un générateur de signaux de balayage en dents de scie (forme d'onde (A), figure 2), de manière à effectuer un balayage sectoriel de la région à traiter, balayage qui passe par le foyer du dispositif émetteur des ondes de traitement.

Les impulsions réfléchies par les structures biologiques sont amplifiées par un récepteur 4 à la sortie duquel est relié un convertisseur analogique-numérique 5.

Un commutateur électronique 6 permet d'aiguiller la sortie du convertisseur 5 sur l'une ou l'autre de deux mémoires 61 et 62. La commutation s'effectue à chaque balayage, le commutateur 6 étant, à cet effet, relié à une sortie appropriée du générateur de balayage 3.

L'adressage de l'écriture dans chaque mémoire est commandé, de façon connue en soi, en fonction de la position angulaire du faisceau émis par la sonde et du temps écoulé depuis le début de chaque émission, pour qu'une image complète de la région traitée soit inscrite dans l'une des deux mémoires à chaque balayage.

La lecture des mémoires s'effectue de façon également connue en soi et les signaux correspondant sont appliqués par l'intermédiaire d'un commutateur 71 qui inverse la connexion entre ses entrées $E_1$, $E_2$ et ses sorties $S_1$, $S_2$ à chaque balayage (étant, à cet effet, relié à la sortie appropriée du générateur 3), à un soustracteur numérique 7.

En l'absence de l'inversion, le soustracteur, qui prend la différence entre les digits série qui définissent les points successifs des deux images, effectuerait successivement la soustraction entre l'image actuelle et l'image précédente, puis entre l'image précédente et l'image actuelle, etc..., d'où la nécessité de l'inversion pour obtenir, à chaque balayage, la soustraction entre l'image actuelle et l'image précédente.

La sortie du soustracteur 7 est appliquée à un convertisseur numérique-analogique 72, qui fournit une tension de modulation de brillance du tube cathodique du dispositif de visualisation 8.

La sortie $S_2$ est par ailleurs reliée à un second convertisseur numérique-analogique 73 et un potentiomètre 74 permet de mélanger, dans une proportion réglable, la tension de sortie du convertisseur 72, qui correspond à l'image différentielle et la tension de sortie du convertisseur 73, qui correspond à la dernière image enregistrée.

L'opérateur a ainsi la possibilité d'observer, soit l'image classique de la région traitée, ce qui lui permettra d'effectuer une première identification des structures, soit l'image différentielle, ce qui lui permettra d'observer l'évolution des structures au cours du traitement.

Comme le traitement s'effectue avec des puissances de crête élevées, la formation des images ne peut avoir lieu pendant l'émission des ondes de traitement, l'énergie de ces ondes, réfléchie par les structures, étant suffisante pour éblouir le transducteur échographique. Cet éblouissement peut se prolonger une ou plusieurs microsecondes après la fin de l'émission. Il est donc nécessaire d'effectuer l'émission par trains d'ondes séparés par des temps morts (forme d'onde (B), figure 2) un peu supérieurs à la durée d'un balayage échographique, lequel dure par exemple, 1/20 sec. et de synchroniser ce dernier avec l'émission.

Il est évidemment nécessaire, par ailleurs, que la cadence de formation des images soit suffisante pour que les mouvements naturels des tissus sous l'influence de la respiration n'introduisent pas de trop grandes différences entre deux images successives, ce qui aurait pour effet de masquer l'effet différentiel lié à la modification des structures due au traitement. A titre d'exemple, on pourra choisir la durée du temps d'émission de façon à obtenir une image tous les 0,5 sec. au moins. Ceci implique que la puissance de crête de l'émission de traitement soit suffisante pour qu'une destruction significative des cellules de la cible se produise en quelques dixièmes de seconde.

La figure 2 représente en (C) les intervalles de temps d'écriture dans la mémoire 61, en (D) les intervalles de temps d'écriture dans la mémoire 62, en (E) les intervalles de lecture de la mémoire 61, en (F) les intervalles de lecture dans la mémoire 62, en (G) et (H), les sorties $S_1$ et $S_2$ correspondantes. Les numéros des images en mémoire ont été indiqués. On voit ainsi que l'image précédente est toujours soustraite de l'image actuelle.

Revenant à la figure 1, on a représenté en outre l'émetteur 10 de l'onde de traitement qui excite le transducteur de puissance T, représenté symboliquement sous la forme d'une coupelle sphérique sur laquelle sont déposés des éléments piézoélectriques. Ce transducteur est avantageusement réalisé conformément aux indications du brevet français No 84 06877 et la sonde 1, bien que figurée à part, sera en pratique solidarisée à la coupelle et disposée en son centre et orientée suivant l'axe de celle-ci suivant les indications dudit brevet.

Par ailleurs, on a représenté à la figure 1 des organes qui ne sont pas utilisés dans le mode d'exécution de l'invention décrit jusqu'ici, mais seulement dans la variante qui va maintenant être décrite.

Les organes comprennent un diviseur de fréquence à taux de division réglable 9, une porte ET 11, une mémoire 12, un dispositif de visualisation 13 et un commutateur 14.

Lorsque le commutateur 14 est en position a, le diviseur 9 est connecté au générateur de balayage 3, lequel est agencé pour fournir un signal de synchronisation lorsque l'axe de la sonde passe par le foyer de l'émetteur de l'onde de traitement (centre de la sphère dont la coupelle T constitue une portion). Le taux de division du diviseur est alors réglé à une valeur allant de 1 à 5 par exemple.

Il fournit donc tous les N balayages, un signal bref appliqué d'une part à l'émetteur de puissance 10, qu'il va bloquer pendant sa durée d'environ 1 ms, d'autre part, à la porte 11.

Cette porte se trouve ainsi débloquée pendant 1/1000 sec. pour transmettre à la mémoire 12 le signal numérique issu du convertisseur 5.

Celle-ci a aussi le temps d'acquérir l'information correspondant à une ligne de balayage, dont la durée est seulement de l'ordre de 0,2 ms (contre 0,2 à 0,02 sec. pour l'acquisition d'une image complète).

Ainsi, dans cette variante, l'onde de traitement ne sera interrompue que pendant 1 ms seulement pendant la durée même des trains d'ondes de traitement, et ce, toutes les 1/20 sec. par exemple, ce qui n'entraîne qu'une réduction très faible (2 % par exemple) de la puissance moyenne par rapport à celle d'une onde de traitement non interrompue.

La ligne ainsi acquise "au vol", tous les 1 à 5 balayages, sera celle qui passe par le foyer. Il s'agit d'échographie A et l'information recueillie dans une seule direction est suffisante. On pourrait d'ailleurs obtenir un résultat équivalent en immobilisant la sonde suivant une direction déterminée traversant la cible.

Les échos stockés dans la mémoire 12 sont lus à une cadence de 50 Hz par exemple et affichés en permanence sur l'écran du dispositif 13.

Cette cadence de lecture donne un bon confort visuel.

On notera que c'est ici l'oeil de l'opérateur qui effectue l'équivalent de la soustraction numérique des informations recueillies pendant le traitement, à une cadence telle qu'il puisse apprécier les changements d'amplitude de l'échogramme.

Il doit être bien compris que l'utilisation d'une sonde d'échographie B pour obtenir des échogrammes du type A présente l'avantage de permettre d'utiliser la même sonde en échographie B avant le traitement d'une cible (ce terme désignant ici une partie de la tumeur ayant une dimension identique à celle de la tache focale et le traitement complet de celle-ci consistant à focaliser le faisceau successivement sur les différentes cibles qui la constituent), pour localiser ladite cible conformément aux procédés décrits dans le brevet français No 84 06877 susvisé. Une relocalisation peut même être effectuée avec la même sonde, utilisée en échographie B, pendant le traitement d'une cible, en interrompant celui-ci pendant un temps suffisant pour obtenir une image (soit 1/20 de sec.).

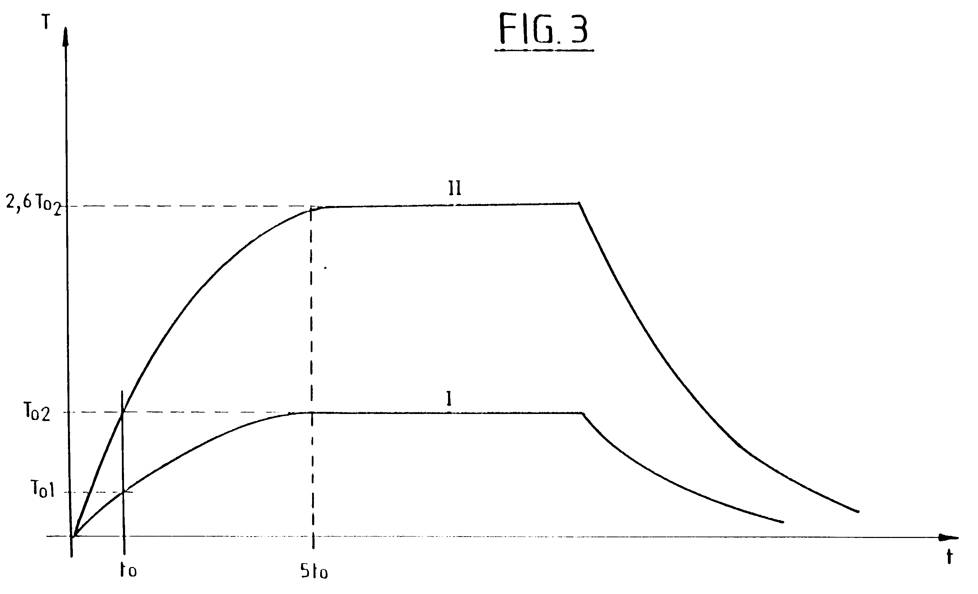

Les autres agencements de l'appareil qui permettent l'hyperthermie ultrarapide vont maintenant être expliqués en se référant à la figure 3.

Celle-ci représente, pour une source calorifique de faible volume, (ici, tache focale de faible diamètre), la montée en température T expérimentalement constatée de la zone soumise aux ondes ultrasonores, en fonction du temps d'application de celles-ci, pour deux valeurs différentes de la puissance appliquée (courbes I et II).

On voit que la montée en température est linéaire pendant une durée $t_o$ qui est sensiblement la même sur les deux courbes, mais correspond à des températures différentes, $T_{o1}$ et $T_{o2}$ respectivement. Les courbes s'infléchissent ensuite pour atteindre un palier au bout d'un temps identique environ égal à $5\,t_o$. La température du palier est environ $3\,T_{o1}$ pour la première courbe, $2,6\,T_{o2}$ pour la seconde.

On constate que $t_o$ est indépendant de la puissance appliquée, et d'autant plus faible que le diamètre de la tache focale est plus réduit. Dans les conditions expérimentales qui ont abouti aux courbes représentées, $t_o$ = 0,5 sec.

Suivant un mode d'exécution préféré de l'invention, on agence l'appareil pour que la destruction totale des cellules soit obtenue pendant un temps au plus égal à $t_o$.

La Demanderesse a constaté qu'il en résulte un endommagement minimal des tissus de la région qui entoure la cible.

Ce résultat expérimental peut s'expliquer par le fait que, dans la région linéaire de la courbe, les pertes par diffusion sont négligeables devant l'apport en calories. Au-delà, entre le point d'inflexion de la courbe, lesdites pertes deviennent proportionnelles au gradient de température entre la cible et les tissus voisins, augmentant ainsi rapidement, jusqu'à devenir égale à l'apport de calories (au palier). Lorsque l'apport de calories cesse, la température de la cible décroît suivant une courbe d'allure exponentielle et atteint une valeur pour laquelle il n'y a sensiblement plus de destruction au bout d'un temps de l'ordre de 3 à 6 $t_o$ dans les conditions expérimentales considérées. Ce temps (ici de l'ordre de 1,5 à 3 sec.) définira sensiblement celui de l'interruption préférentielle entre deux trains successifs d'ondes de traitement, qui permet que les trains successifs agissent tous dans des conditions de pertes minimales par diffusion.

Il en résulte, qu'à la figure 2, où la durée du train est un peu inférieure à 0,5 sec., valeur choisie parce qu'elle correspond à $t_o$, les trains successifs, séparés ici par des intervalles de 0,05 sec. seulement, seront de préférence appliqués à des cibles différentes dans la tumeur, un seul train suffisant pour comparer l'état de réflexivité de chaque cible avant et après son application.

Il est connu par ailleurs que le temps de destruction t des cellules tumorales est proportionnel à la température T à laquelle elles sont soumises, à partir d'une valeur $T_i$ de celle-ci qui est par exemple de 43°C. Pour T = 58°C, t est de l'ordre de 0,5 sec., si bien que l'appareil sera agencé pour obtenir une température de 58°C dans la cible. Le temps d'application t est sensiblement divisé par 2 pour chaque élévation de température de 1°C au-dessus de 43°C, si bien qu'il est par exemple divisé par 1000 quand on passe de 50° à 60°C.

Pour obtenir cette élévation de la température de la cible à environ 20°C au-dessus de la normale en 0,5 sec., soit une vitesse d'échauffement de 40°C/sec., les paramètres suivants sont pris en considération : tout d'abord, la distance focale est imposée par la profondeur de la cible. A titre d'exemple, l'appareil sera agencé pour obtenir trois valeurs de la distance focale, à savoir 4 à 15 cm (pour la destruction de tumeurs profondes), 3 à 4 cm (tumeurs à profondeur intermédiaire), 1 à 1,5 cm (traitement de l'oeil par exemple).

Pour chaque distance focale, la fréquence de travail sera déterminée pour que la concentration globale de l'énergie ultrasonore sur la tache focale soit maximale.

L'expérience montre en effet que c'est lorsque cette première condition est réalisée que, pour une puissance d'émission donnée, les risques d'endommagement des tissus voisins sont minimisés, tandis que la densité d'énergie au niveau de la tache focale est maximale.

Cette constatation peut s'expliquer si l'on considère que cette concentration globale est le produit kg/ka, ka étant un facteur d'atténuation d'autant plus grand que la fréquence est plus élevée (l'expérience montre que l'atténuation est de l'ordre de 1°C par cm de trajet et par MHz) et kg étant égal à

$$\left(\frac{D_o}{d_o}\right)^2 ,$$

$D_o$ et $d_o$ étant les diamètres respectifs de la surface émettrice et de la tache focale.

Le facteur de concentration géométrique kg correspond au rapport des intensités sur la source et sur la tache focale qui serait obtenu, en l'absence d'atténuation sur le trajet du faisceau, et c'est donc le facteur ka qui doit être minimisé si l'on veut éviter les pertes d'énergie dans les tissus traversés, lesquelles ne correspondent d'ailleurs que pour un faible pourcentage à de l'énergie absorbée par les tissus, et qui est la seule à être transformée en chaleur.

La Demanderesse a calculé les valeurs de la concentration globale et de l'énergie résiduelle dans la tache focale, ainsi que de la vitesse d'échauffement de la cible, en fonction de la distance focale, du diamètre d'entrée du faisceau dans les tissus et de la fréquence, pour un angle d'ouverture constant en faisceau et une puissance émise de 1 Kw.

Ces calculs montrent que l'énergie résiduelle croît en raison inverse de la fréquence, mais que l'énergie résiduelle maximum ne correspond pas à la concentration globale maximale, donc à l'échauffement le plus rapide.

Cette constatation peut s'expliquer par le fait que la fréquence agit en sens inverse sur ka et la vitesse d'échauffement d'une part, sur kg d'autre part.

A titre d'exemple, pour une énergie émise de 1 Kw, des distances focales respectives de 10 et 12 cm, avec des diamètres d'entrée dans les tissus de 10 et 12 cm respectivement, la fréquence optimale ainsi relevée est de 1 MHz et donne des vitesses d'échauffement de 33,97 et 21,43°C par sec. respectivement. La puissance devrait donc théoriquement être égale à 1,2 et 1,9 Kw respectivement pour obtenir la vitesse d'échauffement de 40°C/sec. souhaitée. A titre de sécurité, on travaillera à une puissance sensiblement supérieure à ces valeurs, de l'ordre de 10 Kw par exemple, pour tenir compte du fait que la température de 58°C ne sera atteinte qu'à la fin de chaque impulsion du train d'ondes de traitement.

Toutes choses égales par ailleurs, pour des distances focales de 3 et 5 cm et des diamètres d'entrées respectives de 3 et 5 cm, les fréquences optimales seront de 3 et 1,5 MHz respectivement, et les vitesses d'échauffement correspondantes, de 384,89 et 135,91°C/sec. Les puissances thermiques théoriquement nécessaires seront alors sensiblement inférieures à 1 Kw. Cependant, par mesure de sécurité, on prendra des puissances comprises entre 2 et 5 Kw.

Pour une distance focale de 1,5 cm, la fréquence optimale sera de 6 MHz et la valeur d'échauffement de 1539,57°C, ce qui conduira en pratique à prendre une puissance de l'ordre de quelques centaines de watts.

D'une façon générale, les puissances de crête utilisées en hyperthermie ultrarapide, surtout si l'on souhaite pouvoir traiter les tumeurs profondes, exigent que des mesures particulières soient prises dans la réalisation de l'appareil. Il faudra notamment utiliser des céramiques piézoélectriques capables de supporter de telles puissances de crête et de se refroidir rapidement. Des dispositifs de refroidissement pourront être nécessaires. L'alimentation du générateur électrique pourra requérir l'emploi de sources auxiliaires.

On notera que les valeurs optimales de puissance indiquées ne devront pas être dépassées de manière excessive si l'on veut éviter le risque de lésions des tissus voisins. En effet, on a pu montrer que le diamètre de la zone à l'intérieur de laquelle l'élévation de température qui résulte de la diffusion des calories accumulées dans la région focale à l'instant où cesse l'application de la puissance reste suffisante pour détruire les cellules relativement rapidement croît comme la racine carrée de l'élévation de la température dans la région focale, élévation elle-même proportionnelle à la puissance.

On notera enfin qu'aux puissances indiquées, les ondes acoustiques subissent une transformation progressive notable au cours de leur propagation, dont le résultat est l'apparition de composants de fréquences plus élevées que celles de l'onde de départ.

Ces composants à fréquence élevée sont plus fortement absorbés par les tissus et ont donc un effet thermique plus important.

Les fréquences et les puissances retenues permettent aux ondes de traverser avec peu d'altération les premières couches de tissus et de produire un effet thermique au niveau de la tache focale.

Par ailleurs, ces ondes ont un effet mécanique qui s'ajoute à leur effet thermique pour augmenter l'efficacité du traitement.

**Revendications**

1. Appareil d'hyperthermie ultrasonore extracorporelle, permettant l'émission focalisée de trains d'ondes ultrasonores à différentes profondeurs, caractérisé en ce que les dits trains d'ondes ultrasonores présentent une fréquence comprise entre 0,5 et 10 MHz et des puissances électriques de crête comprises entre 10 kW et quelques centaines de watts, les paramètres qui définissent la concentration globale du faisceau ultrasonore dans la tache focale et la puissance étant déterminés pour que, quelle que soit la profondeur et la nature des tissus, le temps de l'émission de traitement soit de l'ordre de celui qui permet d'atteindre une température de destruction de la cible pendant la partie linéaire de la courbe d'élévation de température de celle-ci en fonction du temps.

2. Appareil selon la revendication 1, caractérisé en ce que la fréquence ainsi que le diamètre de la surface émettrice dudit appareil et celui de la tache focale sont déterminés en fonction de la nature et de la profondeur de la cible, pour que le facteur de concentration globale du faisceau soit maximal et la puissance est alors déterminée pour une valeur donnée de la fréquence et des diamètres sélectionnés, pour que la destruction de la cible s'effectue à une température voisine de 58°C, en un temps compris entre 0,5 et 3 secondes.

3. Appareil selon la revendication 1 ou 2, caractérisé en ce que les trains d'ondes successivement appliqués à une même cible sont séparés par des intervalles de temps de l'ordre de 1,5 à 3 secondes.

4. Appareil selon l'une des revendications 1 à 3, caractérisé par une puissance électrique d'émission de l'ordre de 10 kW pour des distances focales de 10 et 12 cm et une fréquence optimale de 1 MHz, une puissance électrique d'émission comprise entre 2 et 5 kW pour des distances focales de 3 et 5 cm et des fréquences optimales de 3 et 1,5 MHz respectivement, et une puissance électrique d'émission de l'ordre de quelques centaines de watts pour une distance focale de 1,5 cm et une fréquence optimale de 6 MHz.

5. Appareil selon l'une des revendications 1 à 4, comportant des moyens d'examen échographique des altérations de la cible au cours du traitement, caractérisé en ce que lesdits moyens effectuent une échographie B de la cible et sont agencés pour comparer deux images successives prises avant et après au moins un train d'ondes de traitement.

6. Appareil selon la revendication 5, caractérisé par des moyens de mémoriser des images successives sous forme d'information numérique et de former une image différentielle par soustraction point par point de l'information mémorisée.

7. Appareil selon la revendicatif 6, caractérisé par des moyens de superposer à l'image différentielle l'une des images mémorisées.

8. Appareil selon l'une des revendications 1 à 7, comportant des moyens d'examen échographique des altérations de la cible au cours du traitement, caractérisé en ce que lesdits moyens pour effectuer une échographie A de la cible utilisent une sonde à balayage travaillant en échographie B et comportent la saisie au vol des échos lorsque le faisceau échographique passe par une direction présélectionnée, pendant des interruptions extrêmement brèves de trains d'ondes de traitement.

9. Procédé de détermination des paramètres de réglages d'un appareil d'hyperthermie ultrasonore extracorporelle, comprenant les étapes consistant à:

- déterminer la distance focale à partir de la profondeur de la dite cible;
- choisir la fréquence des trains d'ondes ultrasonores dans une plage entre 0,5 et 10 MHz pour que la concentration globale de l'énergie ultrasonore soit maximale sur la tache focale, à la distance focale déterminée;
- déterminer expérimentalement la durée de la partie linéaire de la montée en température;
- choisir la puissance électrique de crête dans une plage entre quelques centaines de watts et 10 kW, pour atteindre une température de destruction de la cible pendant la partie linéaire de la courbe d'élévation de température.

10. Procédé selon la revendication 9, caractérisé en ce que la puissance électrique de crête est déterminée pour une valeur donnée de la fréquence et des diamètres sélectionnés, pour que la destruction de la cible s'effectue à une température voisine de 58°C, en un temps compris entre 0,5 et 3 secondes.

11. Procédé selon la revendication 9 ou 10, caractérisé en ce que les trains d'ondes successivement appliqués à une même cible sont séparés par des intervalles de temps de l'ordre de 1,5 à 3 secondes.

12. Procédé selon l'une des revendications 9 à 11, caractérisé en ce que l'on détermine une puissance électrique d'émission de l'ordre de 10 kW pour des distances focales de 10 et 12 cm et une fréquence optimale de 1 MHz, une puissance électrique d'émission comprise entre 2 et 5 kW pour des distances focales de 3 et 5 cm et des fréquences optimales de 3 et 1,5 MHz respectivement, et une puissance électrique d'émission de l'ordre de quelques centaines de watts pour une distance focale de 1,5 cm et une fréquence optimale de 6 MHz.

## Claims

1. An extracorporeal ultrasound hyperthermia treatment device adapted to enable the focussed emission of ultrasound wave trains at varying depths, characterized in that said ultrasound wave trains are of a frequency comprised between 0.5 and 10 MHz with electrical power peaks of between 10 kW and a few hundred watts, the parameters defining overall ultrasound beam concentration in the focal spot and power being determined whereby, irrespective of the depth and nature of the tissue, the treatment emission time is of the order of the time needed to reach a temperature at which the target is destroyed during the linear portion of the curve showing target temperature increase as a function of time.

2. Device according to claim 1, characterized in that the frequency and the diameter of the emitting surface of said device and the diameter the focal spot are determined according to the nature and the depth of the target so that the overall concentration factor of the treatment beam is maximized and the power is then determined for a given value of the frequency and for selected diameters, whereby target destruction takes place at a temperature in the vicinity of 58°C in a time period comprised between 0.5 and 3 seconds.

3. Device according to claim 1 or 2, characterized in that the wave trains successively applied to a given target are separated by time periods of the order of 1.5 to 3 seconds.

4. Device according to one of claims 1 to 3 characterized by an emission power of the order of 10 kW for focal lengths of 10 and 12 cm and an optimum frequency of 1 MHz, an electrical emission power of between 2 and 5 kW for focal lengths of 3 and 5 cm and optimum frequencies of 3 and 1.5 MHz, respectively, and an electrical emission power of the order of several hundreds of watts for a focal length of 1.5 cm and an optimum frequency of 6 MHz.

5. Device according to one of claims 1 to 4, comprising means for echographic examination of changes occurring at the target during treatment, characterized in that said means execute type B echography of the target and are adapted to compare two consecutive images taken before and after at least one treatment wave train.

6. Device according to claim 5, characterized by means for storing successive images in the form of digital information and for forming a differential image by point-by-point subtraction of the stored information.

7. Device according to claim 6, characterized by means for superimposing one of the stored image on the differential image.

8. Device according to one of claims 1 to 7, comprising means for carrying out echographic examination of changes in the target during treatment, characterized in that said echographic examination means carry out type A echography using a scanning probe that performs type B echography and including on-the-fly echo capture when the echographic examination beam passes along a predetermined direction and during extremely short interruptions of the treatment wave trains.

9. Method for determining the correct setting parameters of an extracorporeal ultrasound hyperthermia device, comprising the steps of:

- determining focal length on the basis of the depth of said target;
- selecting the frequency for the ultrasound wave trains in a range of between 0.5 and 10 MHz so that the overall ultrasound energy concentration is a maximum at the focal spot, at the determined focal length;
- experimentally determining the duration of the linear portion of the rise in temperature;
- selecting peak electrical power in a range comprised between several hundreds of watts and 10 kW in order to reach a target destruction temperature during the linear portion of the temperature rise curve.

10. Method according to claim 9, characterized in that the peak electrical power is determined for a given value of the frequency and for selected diameters, whereby target destruction takes place at a temperature in the vicinity of 58°C in a time period comprised between 0.5 and 3 seconds.

11. Method according to claim 9 or 10, characterized in that the wave trains successively applied to a given target are separated by time periods of the order of 1.5 to 3 seconds.

12. Method according to one of claims 9 to 11, characterized in that it comprises determining an emission power of the order of 10 kW for focal lengths of 10 and 12 cm and an optimum frequency of 1 MHz, an electrical emission power of between 2 and 5 kW for focal lengths of 3 and 5 cm and optimum frequencies of 3 and 1.5 MHz, respectively, and an electrical emission power of the order of several hundreds of watts for a focal length of 1.5 cm and an optimum frequency of 6 MHz.

**Patentansprüche**

1. Extrakorporales Ultraschallhyperthermiegerät, das die Emission von auf unterschiedliche Tiefen fokussierten Ultraschallwellenzügen gestattet, dadurch gekennzeichnet, daß die genannten Ultraschallwellenzüge eine Frequenz zwischen 0,5 und 10 MHz und elektrische Spitzenleistungen zwischen 10 kW und einigen hundert Watt aufweisen, wobei die Parameter, die die globale Konzentration des Ultraschallstrahls im Brennpunkt und die Leistung definieren, so bestimmt werden, daß, was immer die Tiefe und die Art der Gewebe sei, die Emissionszeit bei der Behandlung in der Größenordnung derjenigen liegt, die es gestattet, eine das Target zerstörende Temperatur während des linearen Teils der Temperatur-Anstiegskurve desselben als Funktion der Zeit zu erreichen.

2. Gerät nach Anspruch 1, dadurch gekennzeichnet, daß die Frequenz sowie der Durchmesser der emittierenden Fläche des Gerätes und derjenige des Brennpunkts in Abhängigkeit von der Art und der Tiefe des Targets so bestimmt werden, daß der Faktor der globalen Konzentration des Strahls maximal ist, und die Leistung dann für einen gegebenen Wert der Frequenz und der gewählten Durchmesser so bestimmt wird, daß die Zerstörung des Targets bei einer Temperatur in der Nähe von 58 °C innerhalb einer Zeit zwischen 0,5 und 3 Sekunden erfolgt.

3. Gerät nach Anspruch 1 oder 2, dadurch gekennzeichnet, daß die nacheinander auf dasselbe Target abgestrahlten Wellenzüge durch Zeitintervalle in der Größenordnung von 1,5 bis 3 Sekunden voneinander getrennt sind.

4. Gerät nach einem der Ansprüche 1 bis 3, gekennzeichnet durch eine elektrische Emissionsleistung in der Größenordnung von 10 kW für Brennweiten von 10 und 12 cm und eine optimale Frequenz von 1 MHz, eine elektrische Emissionsleistung zwischen 2 und 5 kW für Brennweiten von 3 und 5 cm und optimale Frequenzen von 3 bzw. 1,5 MHz, und eine elektrische Emissionsleistung in der Größenordnung von einigen hundert Watt für eine Brennweite von 1,5 cm und eine optimale Frequenz von 6 MHz.

5. Gerät nach einem der Ansprüche 1 bis 4, mit Mitteln zur echographischen Untersuchung von Veränderungen des Targets im Laufe der Behandlung, dadurch gekennzeichnet, daß diese Mittel eine Echographie B des Targets bewirken und dazu ausgebildet sind, zwei aufeinanderfolgend vor und nach wenigstens einem Behandlungswellenzug aufgenommene Bilder zu vergleichen.

6. Gerät nach Anspruch 5, gekennzeichnet durch Mittel zum Speichern der aufeinanderfolgenden Bilder in der Form numerischer Information und zum Bilden eines Differenzbildes durch punktweise Subtraktion der gespeicherten Information.

7. Gerät nach Anspruch 6, gekennzeichnet durch Mittel zum Überlagern des Differenzbildes mit einem der gespeicherten Bilder.

8. Gerät nach einem der Ansprüche 1 bis 7, mit Mitteln zur echographischen Untersuchung von Veränderungen des Targets im Laufe der Behandlung, dadurch gekennzeichnet, daß diese Mittel zum Bewirken einer Echographie A des Targets eine mit Echographie B arbeitende Abtastsonde verwenden und die fliegende Erfassung von Echos umfassen, wenn der echographische Strahl eine vorgewählte Richtung durchläuft, während extrem kurzer Unterbrechungen von Behandlungswellenzügen.

9. Verfahren zur Bestimmung von Einstellparametern eines extrakorporalen Ultraschallhyperthermiegerätes, mit den Schritten:

   - Bestimmen der Brennweite anhand der Tiefe des genannten Targets,
   - Wählen der Frequenz von Ultraschallwellenzügen in einem Bereich zwischen 0,5 und 10 MHz, so daß die globale Konzentration der Ultraschallenergie im Brennpunkt maximal wird, bei der bestimmten Brennweite,
   - experimentelles Bestimmen der Dauer des linearen Teils des Temperaturanstiegs,
   - Wählen der elektrischen Spitzenleistung in einem Bereich zwischen einigen hundert Watt und 10 kW, um eine das Target zerstörende Temperatur während des linearen Teils der Temperaturanstiegskurve zu erreichen.

10. Verfahren nach Anspruch 9, dadurch gekennzeichnet, daß die elektrische Spitzenleistung für einen gegebenen Wert der Frequenz und der gewählten Durchmesser so bestimmt wird, daß die Zerstörung des Targets bei einer Temperatur in der Nähe von 58 °C innerhalb einer Zeit zwischen 0,5 und 3 Sekunden erfolgt.

11. Verfahren nach Anspruch 9 oder 10, dadurch gekennzeichnet, daß die nacheinander auf dasselbe Target abgegebenen Wellenzüge durch Zeitintervalle in der Größenordnung von 1,5 bis 3 Sekunden voneinander getrennt sind.

12. Verfahren nach einem der Ansprüche 9 bis 11, dadurch gekennzeichnet, daß man eine elektrische Emissionsleistung in der Größenordnung von 10 kW für Brennweiten von 10 und 12 cm und eine optimale Frequenz von 1 MHz bestimmt, eine elektrische Emissionsleistung zwischen 2 und 5 kW für Brennweiten von 3 und 5 cm und optimale Frequenzen von 3 bzw. 1,5 MHz bestimmt und eine elektrische Emissionsleistung in der Größenordnung von einigen hundert Watt für eine Brennweite von 1,5 cm und eine optimale Frequenz von 6 MHz bestimmt.

FIG.1

FIG. 2

EP 0 468 847 B1

FIG. 3

EP 0 468 847 B1